# NEUE EUROPÄISCHE PATENTSCHRIFT

(11) **EP 1 412 339 B2**
(45) Veröffentlichungstag und Bekanntmachung des Hinweises auf die Entscheidung über den Einspruch: **16.10.2013**
(45) Hinweis auf die Patenterteilung: 02.11.2005
(21) Anmeldenummer: 02767229.4
(22) Anmeldetag: 18.07.2002
(51) Int. Cl.: C07D 301/12

(54) **VERFAHREN ZUR HERSTELLUNG VON PROPYLENOXID**
METHOD FOR PRODUCING PROPYLENE OXIDE
PROCEDE DE PREPARATION D'OXYDE DE PROPYLENE

(30) Priorität: 19.07.2001 DE 10135296
(43) Veröffentlichungstag der Anmeldung: 28.04.2004
(73) Patentinhaber: BASF SE, 67056 Ludwigshafen (DE)
(72) Erfinder: TELES, Joaquim, Henrique, 67166 Otterstadt (DE); REHFINGER, Alwin, 67112 Mutterstadt (DE); BERG, Anne, 2170 Merksem (BE); RUDOLF, Peter, 68526 Ladenburg (DE); RIEBER, Norbert, 68259 Mannheim (DE); BASSLER, Peter, 68519 Viernheim (DE)
(74) Vertreter: Altmann, Andreas
(86) Internationale Anmeldenummer: PCT/EP2002/008022
(87) Internationale Veröffentlichungsnummer: WO 2003/008401

(56) Entgegenhaltungen:
- EP-A- 0 719 768
- DE-A- 19 623 608
- DE-A- 19 623 611

## Beschreibung

Die vorliegende Erfindung betrifft ein Verfahren, in welchem Propylenoxid aus einem Hydroperoxid, bevorzugt Wasserstoffperoxid, und Propen hergestellt wird, wobei ein Teil des nicht-umgesetzten Propens durch eine geeignete Vorgehensweise wiedergewonnen und in eine Umsetzung von Propen mit Hydroperoxid zurückgeführt wird.

Grundsätzlich ist es bei Verfahren, in denen Gemische aus einem Alken und Sauerstoff anfallen, in zahlreichen Verfahrensführungen erwünscht, das Alken aus diesem Gemisch ganz oder teilweise abzutrennen und gegebenenfalls aus prozessökonomischen Gründen wieder in eine Verfahrensstufe rückzuführen. Ein Problem, das sich bei diesen Verfahren stellt, ist die Entstehung von zündfähigen Gemischen, die es selbstverständlich aus sicherheitstechnischen Aspekten unter allen Umständen zu vermeiden gilt.

Ein Verfahren, bei dem dieses Problem auftritt, ist die Herstellung von Propylenoxid, einem wichtigen Zwischenprodukt der chemischen Industrie, ausgehend von Propen und Wasserstoffperoxid. Im Zuge der Produktaufbereitung wird in diesem Verfahren bevorzugt nicht-umgesetztes Propen aus dem Austrag der Epoxidierung abgetrennt und erneut in das Verfahren als Edukt rückgeführt. Häufig wird das Propen bei der Abtrennung aus dem Austrag von anderen leicht siedenden und im Austrag ebenfalls vorhandenen Komponenten, unter anderem Sauerstoff, begleitet. Dabei wird der Sauerstoff meist in einer Konzentration angereichert, welche zu einem zündfähigen Gemisch aus Propen und Sauerstoff führt. Dieses stellt für den Abtrennungsvorgang ein nicht zu unterschätzendes Sicherheitsrisiko dar.

Um dieses Problem zu lösen, schlägt die EP-B 0 719 768 vor, die Abtrennung von Propen aus dem Leichtsiedegemisch in einer sogenannten Absorptionszone vorzunehmen. In dieser wird ein Inertgas, bevorzugt Methan, bis zu einer solchen Konzentration zugeführt, dass der ebenfalls im Leichtsiedegemisch vorhandene Sauerstoff bis zu einer Konzentration verdünnt ist, bei welcher das Gemisch nicht mehr im zündfähigen Bereich liegt. Weiterhin ist es bei dieser Verfahrensführung notwendig, der Absorptionszone flüssiges Absorptionsmittel zuzuführen, in welchem das Propen aus der Leichtsiedefraktion herausgewaschen wird.
Die WO 01/57009 betrifft ein Verfahren zur katalytischen Epoxidation von Olefinen, bei dem in einer Rektionsstufe das Olefin mit wässrigem Wasserstoffperoxid in einem organischen, wassermischbaren Lösemittel in der Gegenwart eines Titanium-Silikalit Katalysators umgesetzt wird. Der dabei erhaltene Abgasstrom wird mit einer Absorptionseinheit mit demselben Lösemittel in Kontakt gebracht, das bereits in der Reaktionsstufe eingesetzt wurde, wobei ein Lösemittelstrom abgezogen wird, der Olefin und Olefinoxid enthält und ein Abgasstrom enthaltend Sauerstoff verworfen wird.

Demgemäss müssen im eben beschriebenen Verfahren zur Epoxidation von Propen mit Wasserstoffperoxid zwei zusätzliche Komponenten, das Inertgas sowie das flüssige Absorptionsmittel, eingesetzt werden, um nicht-umgesetztes Propen unter Vermeidung der Bildung entflammbarer Gasgemische abzutrennen, bzw. rückzugewinnen.

Folglich ist dieses Verfahren sowohl mit erhöhten Kosten als auch erhöhtem apparativem Aufwand für den Einsatz der zusätzlichen und aus sicherheitstechnischen Gründen bei diesem Verfahren notwendigen Komponenten belastet.

Somit lag der vorliegenden Erfindung die Aufgabe zugrunde, ein Verfahren zur Herstellung von Propylenoxid bereitzustellen, innerhalb dessen es möglich ist, aus einem Sauerstoff und nicht-umgesetztes Propen aufweisenden gasförmigen Gemisch, in einfacher und sicherer Verfahrensweise wenigstens einen Teil des nicht-umgesetzten Propens zurückzugewinnen, um dieses erneut in eine Verfahrensstufe als Edukt einzusetzen.

Demgemäss betrifft die vorliegende Erfindung ein Verfahren zur Herstellung von Propylenoxid, welches wenigstens die folgenden Schritte aufweist:
(a) Propen, gegebenenfalls enthaltend bis zu 10 Gew.-% von Propen verschiedene Kohlenwasserstoffe, wird mit einem Hydroperoxid in einem Lösungsmittel in Anwesenheit eines Zeolith-Katalysators unter Erhalt eines Gemisches (G0) umgesetzt, wobei Gemisch (G0) Propylenoxid, Lösungsmittel, nicht-umgesetztes Propen, nicht-umgesetztes Hydroperoxid und Sauerstoff aufweist,
(b) das Propylenoxid wird aus Gemisch (G0) derart abgetrennt, dass ein Gemisch (G1) erhalten wird, welches nicht-umgesetztes Propen und Sauerstoff aufweist;
(c) das Gemisch (G1) wird unter Erhalt der Gemische (G2) und (G3) mit einem fluiden Medium, welches wenigstens Lösungsmittel aufweist, in Kontakt gebracht,
   wobei das Gemisch (G3) einen Teil des nicht-umgesetzten Propens und Sauerstoff, das Gemisch (G2) Lösungsmittel und Rest-Propen aufweist und
   wobei das abgetrennte, nicht-umgesetzte Propen und Sauerstoff aufweisende Gemisch (G3) ein Verhältnis von Sauerstoff zu Propen aufweist, durch welches das Gemisch (G3) nicht zündfähig ist und
   wobei das Gemisch (G2) wenigstens einer Umsetzung von Propen mit Hydroperoxid zugeführt wird, und wobei das Gemisch (G2) ferner Hydroperoxid enthält, und dass in weiteren, dem Schritt (c) folgenden Schritten (d) und (e) das Gemisch (G2) einer Umsetzung von Propen mit Hydroperoxid wie folgt zugeführt wird:
   (d) das Gemisch (G2) wird unter Erhalt eines Gemisches (G4) mit weiterem Propen versetzt, wobei das Propen unter weitgehender Umsetzung des im Gemisch (G2)
   noch vorhandenen nicht-umgesetzten Hydroperoxids zu Propylenoxid umgesetzt und (e) das Gemisch (G4) räumlich in die Abtrennung gemäß Schritt (b) zurückgeführt wird.

Die Auftrennung eines Gemisches in wenigstens zwei Fraktionen kann im Rahmen der Erfindung grundsätzlich nach sämtlichen geeigneten Methoden erfolgen.

Bevorzugt wird im Rahmen der vorliegenden Erfindung die Auftrennung eines im wesentlichen flüssigen Gemisches mittels wenigstens einer Destillationskolonne erreicht.
Zur ganzen oder teilweisen Abtrennung von wenigstens einer gasförmigen Komponente aus einem im wesentlichen gasförmigen Gemisch wird im Rahmen der vorliegenden Erfindung hingegen bevorzugt eine sogenannte "Absorptionskolonne" eingesetzt.

In dieser wird das die Kolonne durchströmende, im wesentlichen gasförmige Gemisch mit einem fluiden Medium (Absorptionsmittel), in der Regel im Gegenstromverfahren, gewaschen, wobei die gewünschte Gaskomponente ganz oder teilweise von dem Absorptionsmittel absorbiert wird und die Kolonne zusammen mit dem Absorptionsmittel verlässt. Die nicht absorbierte(n) Gaskomponente(n) können die Kolonne durch eine weitere geeignete Vorrichtung verlassen.

Im Rahmen der vorliegenden Erfindung handelt es sich bei der gewünschten, vom fluiden Medium zu absorbierenden Gaskomponente um Propen. Die Menge an Propen, welche durch das fluide Medium (Absorptionsmittel) innerhalb des Gaswaschvorgangs absorbiert wird, kann über alle dem Fachmann für diesen Zweck geeignet erscheinenden Parameter gesteuert werden, beispielsweise über die Menge an Absorptionsmittel oder den Druck in der Absorptionskolonne, welcher in einem Bereich von 0,5 bis 3 bar, bevorzugt im Normaldruckbereich liegt. Auch eine Steuerung über die in der Absorptionskolonne vorherrschende Temperatur, welche in einem Bereich von 0 bis 60 °C, bevorzugt in einem Bereich von 25 bis 40°C liegt, ist möglich.

Im Rahmen der Erfindung ist der zur Steuerung der Menge an Propen, welche durch das fluide Medium (Absorptionsmittel) innerhalb des Gaswaschvorgangs absorbiert wird, bevorzugt eingesetzte Parameter die Menge an eingesetztem Absorptionsmittel.

Im Rahmen der Erfindung wird nur ein Teil des in einem gasförmigen Gemisch enthaltenen Propens, das sogenannte Rest-Propen, durch das Absorptionsmittel innerhalb der wenigstens einen Absorptionskolonne absorbiert, so dass das nach dem Waschvorgang verbleibende gasförmige Gemisch weiterhin Propen aufweist.

Das zur Absorption eingesetzte fluide Medium (Absorptionsmittel) kann im Rahmen der vorliegenden Erfindung grundsätzlich jedes zur Absorption von Propen geeignete flüssige Mittel sein. Dies sind z.B. sämtliche dem Fachmann bekannten und für diesen Zweck geeigneten Lösungsmittel. Demgemäss können beispielsweise folgende Lösungsmittel als Absorptionsmittel eingesetzt werden:
- Wasser,
- Alkohole, bevorzugt niedere Alkohole, weiter bevorzugt Alkohole mit weniger als 6 Kohlenstoffatomen wie beispielsweise Methanol, Ethanol, Propanole, Butanole, Pentanole,
- Diole oder Polyole, bevorzugt solche mit weniger als 6 Kohlenstoffatomen,
- Ether wie beispielsweise Diethylether, Tetrahydrofuran, Dioxan, 1,2-Diethoxyethan, 2-Methoxyethanol,
- Ester wie beispielsweise Methylacetat oder Butyrolacton,
- Amide wie beispielsweise Dimethylformamid, Dimethylacetamid, N-Methylpyrrolidon,
- Ketone wie beispielsweise Aceton,
- Nitrile wie beispielsweise Acetonitril
- oder Gemische aus zwei oder mehr der vorgenannten Verbindungen.

Bevorzugt wird im Rahmen der Erfindung als Absorptionsmittel ein aus einem vorangegangenen Verfahrensschritt hervorgehendes flüssiges Medium eingesetzt. Dieses muss in der Lage sein, einen bestimmten Teil des nicht-umgesetzten Propens aus dem gasförmigen Gemisch (G1), mit welchem es in der Absorptionskolonne in Kontakt gebracht wird, während des Gaswaschvorgangs zu absorbieren. Besonders bevorzugt weist das in Rede stehende fluide Medium wenigstens ein, im erfindungsgemäßen Verfahren eingesetztes Lösungsmittel auf.

Im Rahmen der Erfindung wird in Schritt (c) das Gemisch (G1), welches nicht-umgesetztes Propen und Sauerstoff aufweist, unter Erhalt der Gemische (G2) und (G3) mit einem fluiden Medium (Absorptionsmittel), welches wenigstens Lösungsmittel aufweist, in Kontakt gebracht.

In diesem Verfahrensschritt wird durch das Absorptionsmittel nicht-umgesetztes Propen aus dem Gemisch (G1) entfernt und im Anschluss wieder in das erfindungsgemäße Verfahren zurückgeführt. Die in diesem Schritt entfernte Menge an nicht-umgesetzten Propen ist dadurch begrenzt, dass das verbleibende Gemisch (G3) nicht zündfähig werden darf

Weiterhin ist es aus sicherheitstechnischen Überlegungen heraus wichtig, dass das die Absorptionskolonne über eine geeignete Auslassvorrichtung verlassende Gemisch (G3), welches das nicht durch das Absorptionsmittel absorbierte Propen sowie Sauerstoff enthält, ein Verhältnis von Sauerstoff zu Propen und gegebenenfalls weiteren in (G3) vorhandenen brennbaren Komponenten aufweist, durch welches das Gemisch (G3) nicht zündfähig ist.

Der Begriff "nicht zündfähig" bedeutet im Rahmen der vorliegenden Erfindung, dass die Zusammensetzung des Gemisches (G3) so gewählt werden muss, dass es unter den bei seiner Abtrennung vorliegenden Verfahrensbedingungen eine Zündgrenze aufweist, bei welcher das Gemisch (G3) ohne dass es zündet handhabbar ist, wobei der Begriff "zündfähiges Gemisch" nach der Definition der Berufsgenossenschaft Chemie BGR 104 Teil 1 - Explosionsschutzregeln, Abschnitt B, Punkt 9 "ein Gemisch von Gasen und Dämpfen untereinander oder mit Nebeln oder Stäuben, in dem sich nach erfolgter Zündung eine Reaktion selbständig fortpflanzt" bezeichnet.

Grundsätzlich versteht man unter der Zündgrenze die untere und obere Grenzkonzentration eines brennbaren Gases oder Dampfes in Mischung mit Luft (oder einem anderen, Sauerstoff enthaltenden Gas), zwischen denen das Gas-(Dampf-) Luft-Gemisch durch Erhitzen (Zündtemperatur) oder Funken zur Zündung gebracht werden kann. Die Zündgrenzen sind druck- und temperaturabhängig. Sie werden als Konzentration des brennbaren Gases, Dampfes bzw. Sauerstoffs in Vol.-% oder g/m³ für einen Anfangszustand von 1013 mbar und 20 °C angegeben.

Die Zündgrenze eines Gemisches hängt im wesentlichen von der Zusammensetzung seiner Hauptkomponenten ab. Eine beispielhafte Übersicht bezüglich sicherheitstechnischer Kennzahlen brennbarer Gase und Dämpfe findet sich in folgenden Nachschlagewerken: Coward & Jones, US Bureau of Mines Bull. 503 (1952); Nabert & Schön, Sicherheitstechnische Kennzahlen brennbarer Gase und Dämpfe, Deutscher Eichverlag, Braunschweig (1963).

Demgemäss betrifft die vorliegende Erfindung auch ein Verfahren, wie oben beschrieben, wobei die Konzentration von Sauerstoff in Gemisch (G3) weniger als 12 Vol.-%, bevorzugt weniger als 11 Vol.-% , besonders bevorzugt weniger als 10 Vol.-% beträgt..

Im Rahmen der vorliegenden Erfindung ist es selbstverständlich auch möglich, Propen einzusetzen, welches bis zu 10 Gew.-% Kohlenwasserstoffe, die verschieden von Propen sind, aufweist.

Beispielsweise kann das eingesetzte Propen bis zu 10 Gew.-% Propan, Ethan, Ethylen, Butan oder Butene einzeln oder als Gemisch aus zwei oder mehr davon aufweisen.

Demgemäss betrifft die vorliegende Erfindung auch ein Verfahren, wie oben beschrieben, wobei das eingesetzte Propen bis zu 10 Gew.-% andere Kohlenwasserstoffe aufweist.

Schritt (a) des oben aufgeführten erfindungsgemäßen Verfahrens wird in der Regel in einem sogenannten Hauptreaktor (1), bevorzugt einem Rohrbündelreaktor ausgeführt.

Im Rahmen der Erfindung ist es vorteilhaft, innerhalb des Schritts (a) des erfindungsgemäßen Verfahrens mit einem molaren Verhältnis von Propen zu Hydroperoxid in einem Bereich von 0,85 bis 5, bevorzugt 0,9 bis 2, besonders bevorzugt 0,9 bis 1,2 zu arbeiten.

Grundsätzlich können alle dem Fachmann bekannten Hydroperoxide im Rahmen der Erfindung eingesetzt werden. Details bezüglich der Hydroperoxid-Herstellung bzw. der bevorzugt einsetzbaren Hydroperoxide sind der DE-A 19835907.1 zu entnehmen.

Bevorzugt wird jedoch im Rahmen der vorliegenden Erfindung Wasserstoffperoxid als Hydroperoxid eingesetzt.

Weiterhin bevorzugt liegt der Umsatz an Wasserstoffperoxid im Schritt (a) in einem Bereich von 70 bis 99 %, bevorzugt in einem Bereich von 75 bis 98 %, besonders bevorzugt in einem Bereich von 80 bis 95 %.

Demgemäss betrifft die vorliegende Erfindung auch ein Verfahren, wie oben beschrieben, wobei das Hydroperoxid Wasserstoffperoxid ist und der Umsatz an Wasserstoffperoxid in Schritt (a) im Bereich von 80 bis 95 % liegt.

Im Rahmen der vorliegenden Erfindung können grundsätzlich alle dem Fachmann geeignet erscheinenden Lösungsmittel eingesetzt werden. Beispielsweise können als Lösungsmittel
- Wasser,
- Alkohole, bevorzugt niedere Alkohole, weiter bevorzugt Alkohole mit weniger als 6 Kohlenstoffatomen wie beispielsweise Methanol, Ethanol, Propanole, Butanole, Pentanole,
- Diole oder Polyole, bevorzugt solche mit weniger als 6 Kohlenstoffatomen,
- Ether wie beispielsweise Diethylether, Tetrahydrofuran, Dioxan, 1,2-Diethoxyethan, 2-Methoxyethanol,
- Ester wie beispielsweise Methylacetat oder Butyrolacton,
- Amide wie beispielsweise Dimethylformamid, Dimethylacetamid, N-Methylpyrrolidon,
- Ketone wie beispielsweise Aceton,
- Nitrile wie beispielsweise Acetonitril
- oder Gemische aus zwei oder mehr der vorgenannten Verbindungen eingesetzt werden.

Bevorzugt wird im Rahmen der Erfindung als Lösungsmittel Methanol eingesetzt.

Als Zeolith-Katalysatoren in Schritt (a) können im Rahmen der vorliegenden Erfindung grundsätzlich alle dem Fachmann für eine derartige Umsetzung bekannten Zeolith-Katalysatoren eingesetzt werden.

Bevorzugt werden Zeolithe eingesetzt, welche Eisen-, Titan-, Vanadium-, Chrom-, Niob- oder Zirkonium-haltig sind.

Dabei sind im einzelnen Titan-, Germanium-, Tellur-, Vanadium-, Chrom-, Niob-, Zirkonium-haltige Zeolithe mit Pentasil-Zeolith-Struktur, insbesondere die Typen mit röntgenografischer Zuordnung zur ABW-, ACO-, AEI-, AEL-, AEN-, AET-, AFG-, AFI-, AFN-, AFO-, AFR-, AFS-, AFT-, AFX-, AFY-, AHT-, ANA-, APC-, APD-, AST-, ATN-, ATO-, ATS-, ATT-, ATV-, AWO-, AWW-, BEA-, BIK-, BOG-, BPH-, BRE-, CAN-, CAS-, CFI-, CGF-, CGS-, CHA-, CHI-, CLO-, CON-, CZP-, DAC-, DDR-, DFO-, DFT-, DOH-, DON-, EAB-, EDI-, EMT-, EPI-, ERI-, ESV-, EUO-, FAU-, FER-, GIS-, GME-, GOO-, HEU-, IFR-, ISV-, ITE-, JBW-, KFI-, LAU-, LEV-, LIO-, LOS-, LOV-, LTA-, LTL-, LTN-, MAZ-, MEI-, MEL-, MEP-, MER-, MFI-, MFS-, MON-, MOR-, MSO-, MTF-, MTN-, MTT-, MTW-, MWW-, NAT-, NES-, NON-, OFF-, OSI-, PAR-, PAU-, PHI-, RHO-, RON-, RSN-, RTE-, RTH-, RUT-, SAO-, SAT-, SBE-, SBS-, SBT-, SFF-, SGT-, SOD-, STF-, STI-, STT-, TER-, THO-, TON-, TSC-, VET-, VFI-, VNI-, VSV-, WIE-, WEN-, YUG-, ZON-Struktur sowie zu Mischstrukturen aus zwei oder mehr der vorgenannten Strukturen zu nennen. Denkbar sind für den Einsatz im erfindungsgemäßen Verfahren weiterhin titanhaltige Zeolithe mit der Struktur des ITQ-4, SSZ-24, TTM-1, UTD-1, CIT-1 oder CIT-5. Als weitere titanhaltige Zeolithe sind solche mit der Struktur des ZSM-48 oder ZSM-12 zu nennen.

Bevorzugt werden im Rahmen der vorliegenden Erfindung Ti-Zeolithe mit MFI-, MEL- oder MFI/MEL-Mischstruktur eingesetzt. Als weiterhin bevorzugt sind im einzelnen die Ti-enthaltenden Zeolith-Katalysatoren, die im allgemeinen als "TS-1", "TS-2", "TS-3" bezeichnet werden, sowie Ti-Zeolithe mit einer zu β-Zeolith isomorphen Gerüststruktur zu nennen.

Demgemäss betrifft die vorliegende Erfindung auch ein Verfahren, wie oben beschrieben, wobei der Zeolith-Katalysator ein Titansilikalit-Katalysator, insbesondere ein Titansilikalit-Katalysator der Struktur TS-1 ist.

Weitere Details bezüglich der einsetzbaren Katalysatoren, insbesondere Zeolithe sind der DE-A 1010139.2 zu entnehmen.

Das aus Schritt (a) hervorgehende Gemisch (G0) weist im wesentlichen folgende Komponenten auf: Propylenoxid als gewünschtes Verfahrensprodukt, Lösungsmittel, nicht-umgesetztes Propen, nicht-umgesetztes Hydroperoxid und Sauerstoff.

Als weitere Komponenten kann das Gemisch (G0) auch Wasser, sowie, je nach Propan bzw. Kohlenwasserstoffgehalt des eingesetzten Propens, Propan bzw. weitere Kohlenwasserstoffe aufweisen, wobei die als "Kohlenwasserstoffe" bezeichneten Verbindungen selbstverständlich verschieden von Propen sind.

Aus dem aus Schritt (a) des erfindungsgemäßen Verfahrens resultierenden Gemisch (G0) wird in einem weiteren Schritt (b) Propylenoxid derart abgetrennt, dass ein Gemisch (G1) erhalten wird, welches nicht-umgesetztes Propen und Sauerstoff aufweist.

Die Abtrennung von Propylenoxid aus dem Gemisch (G0) sowie die weitere Aufarbeitung der anderen in (G0) enthaltenden Komponenten erfolgt im Rahmen der vorliegenden Erfindung bevorzugt über die Verfahrensvariante 1.

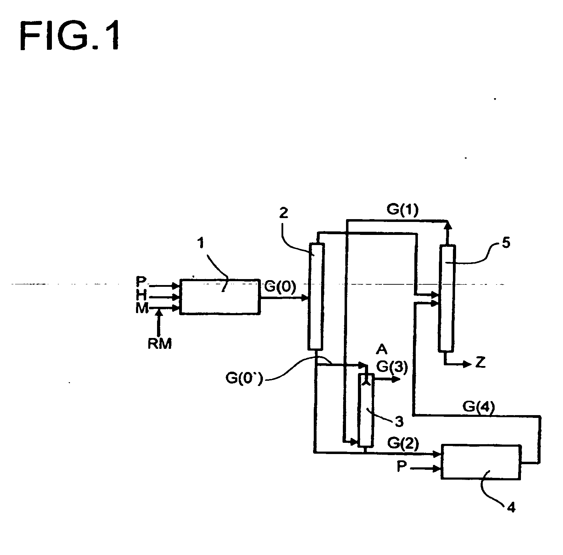

Die Verfahrensführung dieser Variante, welche in Figur 1 schematisch dargestellt ist, wird im Folgenden ausführlich beschrieben.

### Variante 1:

Eine bevorzugte Verfahrensweise (Variante 1, Fig.1) zur Abtrennung des Propylenoxids besteht darin, den Produktaustrag des Hauptreaktors (1) in einer, sich unmittelbar an den Hauptreaktor (1) anschließenden Kolonne (2) in eine Kopf-und eine Sumpffraktion aufzutrennen. Die Sumpffraktion der Kolonne (2), Gemisch (G0'), weist Lösungsmittel, nicht-umgesetztes Hydroperoxid sowie Wasser auf. Die Kopffraktion dieser Kolonne (2) weist im wesentlichen Propylenoxid, nicht-umgesetztes Propen sowie Sauerstoff auf und wird in eine weitere Kolonne (5) überführt.

Gegebenenfalls kann die Kopffraktion dieser Kolonne (2) auch noch kleine Mengen an Lösungsmittel aufweisen. Im Rahmen der Erfindung weist die Kopffraktion der Kolonne (2) bevorzugt kleiner 60 Gew.-% , besonders bevorzugt kleiner 50 Gew.-% an Lösungsmittel auf.

In Kolonne (5) wird das Propylenoxid über die Sumpffraktion abgezogen und gegebenenfalls weiteren Aufarbeitungsschritten, in welchen das Propylenoxid von den weiteren in der Sumpffraktion vorhandenen Komponenten wie Lösungsmittel und Wasser abgetrennt und gereinigt wird, unterworfen.

Im Rahmen der Aufarbeitung der Sumpffraktion aus Kolonne (5) ist es auch möglich, das Lösungsmittel rückzugewinnen und erneut in das Verfahren einzusetzen. Bevorzugt wird das rückgewonnene Lösungsmittel in den Schritt (a) zurückgeführt.

Die Kopffraktion der Kolonne (5), das Gemisch (G1), weist im wesentlichen nicht-umgesetztes Propen und Sauerstoff auf und wird zur weiteren Aufarbeitung in die Absorptionskolonne (3) überführt.

In der Absorptionskolonne (3) wird das gasförmige Gemisch (G1) mit einem Teil oder dem gesamten Gemisch (G0'), der flüssigen Sumpffraktion aus der Kolonne (2), gewaschen. Dabei dient das gesamte oder nur teilweise eingesetzte Gemisch (G0'), welches Lösungsmittel, nicht-umgesetztes Hydroperoxid sowie Wasser aufweist, als Absorptionsmittel für einen Teil des in Gemisch (G1) enthaltenen, nicht-umgesetzten Propens.

Über die Menge des zur Gaswäsche des Gemischs (G1) eingesetzten flüssigen Gemisches (G0') ist es im Rahmen der vorliegenden Erfindung möglich, die Menge des aus dem Gemisch (G1) abzutrennenden nicht-umgesetzten Propens zu steuern.

Die Menge des Gemisches (G0') wird dabei vorzugsweise über geeignete, dem Fachmann bekannte Leitungssysteme, welche beispielsweise wenigstens einen Bypass bzw. Ventilsysteme aufweisen können, festgelegt.

So kann Gemisch (G0') bei Bedarf in zwei oder mehr Fraktionen aufgeteilt werden. Bevorzugt wird dabei wenigstens eine dieser Fraktionen in der Absorptionskolonne als Absorptionsmittel (fluides Medium) zur Gaswäsche des gasförmigen Gemisches (G1) verwendet.

Die nicht der Absorptionskolonne zugeführte Fraktion des Gemisches (G0') kann ganz oder teilweise mit dem die Absorptionskolonne (3) verlassenden flüssigen Gemisch zu einem Gemisch (G2) vereint werden, wobei das die Absorptionskolonne (3) verlassende flüssige Gemisch den durch den Gaswaschvorgang aus Gemisch (G1) absorbierten Teil an Propen (Rest-Propen) aufweist.

Durch den Gaswaschvorgang in der Absorptionskolonne (3) wird somit ein Teil des nicht-umgesetzten Propens aus dem Gemisch (G1) entfernt. Demgemäß weist das aus diesem Verfahrensschritt hervorgehende Gemisch (G2) Rest-Propen sowie die Komponenten des Absorptionsmittels, Lösungsmittel, Wasser und nicht-umgesetztes Wasserstoffperoxid auf.

Das weiterhin aus diesem Verfahrensschritt hervorgehende gasförmige Gemisch (G3), welches die Absorptionskolonne (3) durch eine eigene Auslassvorrichtung verlässt, weist folglich den nicht absorbierten Teil an nicht-umgesetzten Propen sowie Sauerstoff auf.

Die Bedingungen in der Absorptionskolonne (3) müssen dabei so gewählt werden, dass das gasförmige Gemisch (G3), welches die Absorptionskolonne (3) verlässt, nicht-umgesetztes Propen und Sauerstoff in einem Verhältnis aufweist, aufgrund dessen es nicht zündfähig ist.

Weiterhin ist es auch möglich, dass das Gemisch (G3) neben nicht-umgesetztem Propen und Sauerstoff noch kleine Mengen anderer flüchtiger Komponenten enthält.

Das Gemisch (G2) wird wenigstens einer weiteren Umsetzung von Propen mit Hydroperoxid in einem sogenannten Nachreaktor (4), bevorzugt ein Rohrbündelreaktor, zugeführt.

Im Nachreaktor (4) wird Gemisch (G2) erneut mit einer solchen Menge an Propen versetzt, dass das in Gemisch (G2) noch vorhandene nicht-umgesetzte Hydroperoxid unter Erhalt eines Gemisches (G4) weitestgehend mit Propen zu Propylenoxid umgesetzt wird.

Demgemäß weist der Rohaustrag des Nachreaktors (4) neben Propylenoxid, Lösungsmittel, Wasser, nicht-umgesetztes Propen sowie nicht-umgesetztes Hydroperoxid in einem Bereich kleiner 500 ppm auf.

Das Gemisch (G4) kann anschließend zur Abtrennung des gewünschten Produkts Propylenoxid weiter aufgearbeitet werden.

Im Rahmen der Erfindung wird Gemisch (G4) jedoch in Kolonne (5) überführt und dort mit der Kopffraktion aus Kolonne (2) vereint.

Mit dem Gemisch in Kolonne (5) wird wie oben beschrieben verfahren.

Demgemäss betrifft die vorliegende Erfindung ein Verfahren, wie oben beschrieben, wobei das Gemisch (G2) ferner Hydroperoxid enthält und in den weiteren, dem Schritt (c) folgenden Schritten (d) und (e) das Gemisch (G2) einer Umsetzung von Propen mit Hydroperoxid wie folgt zugeführt wird:
(d) das Gemisch (G2) wird unter Erhalt eines Gemisches (G4) mit weiterem Propen versetzt, wobei das Propen unter weitgehender Umsetzung des im Gemisch (G2) noch vorhandenen nicht-umgesetzten Hydroperoxids zu Propylenoxid umgesetzt und
(e) das Gemisch (G4) in die Abtrennung gemäß Schritt (b) zurückgeführt wird.

### Bezugszeichenliste

Figur 1:
   1 Hauptreaktor (1)
   2 Kolonne (2)
   3 Absorptionskolonne (3)
   4 Nachreaktor (4)
   5 Kolonne (5)

### Abkürzungen

- P:: Propen
- H:: H₂O₂
- M:: Methanol
- RM:: Rück- Methanol aus Aufarbeitung
- A:: Abgas
- Z:: Zur weiteren Aufarbeitung

## Patentansprüche

1. Verfahren zur Herstellung von Propylenoxid, welches wenigstens die folgenden Schritte aufweist:
(a) Propen, gegebenenfalls enthaltend bis zu 10 Gew.-% von Propen verschiedene Kohlenwasserstoffe, wird mit einem Hydroperoxid in einem Lösungsmittel in Anwesenheit eines Zeolith-Katalysators unter Erhalt eines Gemisches (G0) umgesetzt, wobei Gemisch (G0) Propylenoxid, Lösungsmittel, nicht-umgesetztes Propen, nicht-umgesetztes Hydroperoxid und Sauerstoff aufweist,
(b) das Propylenoxid wird aus Gemisch (G0) derart abgetrennt, dass ein Gemisch (G1) erhalten wird, welches nicht-umgesetztes Propen und Sauerstoff aufweist;
(c) das Gemisch (G1) wird unter Erhalt der Gemische (G2) und (G3) mit einem fluiden Medium, welches wenigstens Lösungsmittel aufweist, in Kontakt gebracht, wobei das Gemisch (G3) einen Teil des nicht-umgesetzten Propens und Sauerstoff, das Gemisch (G2) Lösungsmittel und Rest-Propen aufweist, und
wobei das abgetrennte nicht-umgesetzte Propen und Sauerstoff aufweisende Gemisch (G3) ein Verhältnis von Sauerstoff und Propen aufweist, durch welches das Gemisch (G3) nicht zündfähig ist und
wobei das Gemisch (G2) wenigstens einer Umsetzung von Propen mit Hydroperoxid zugeführt wird,
und wobei das Gemisch (G2) ferner Hydroperoxid enthält, und dass in weiteren, dem Schritt (c) folgenden Schritten (d) und (e) das Gemisch (G2) einer Umsetzung von Propen mit Hydroperoxid wie folgt zugeführt wird:
(d) das Gemisch (G2) wird unter Erhalt eines Gemisches (G4) mit weiterem Propen versetzt, wobei das Propen unter weitgehender Umsetzung des im Gemisch (G2) noch vorhandenen nicht-umgesetzten Hydroperoxids zu Propylenoxid umgesetzt und
(e) das Gemisch (G4) räumlich in die Abtrennung gemäß Schritt (b) zurückgeführt wird.

2. Verfahren nach Anspruch 1, **dadurch gekennzeichnet, dass** die Konzentration von Sauerstoff im Gemisch (G3) weniger als 12 Vol.-% beträgt.

3. Verfahren nach einem der Ansprüche 1 oder 2, **dadurch gekennzeichnet, dass** das Hydroperoxid Wasserstoffperoxid ist und der Umsatz an Wasserstoffperoxid in (a) im Bereich von 80 bis 95 % liegt.

4. Verfahren nach einem der Ansprüche 1 bis 3, **dadurch gekennzeichnet, dass** der Zeolith-Katalysator ein Titansilikalit-Katalysator, insbesondere ein TitansilikalitKatalysator der Struktur TS-1 ist.

## Claims

1. A process for preparing propylene oxide, which comprises at least the following steps:
(a) propene, optionally containing up to 10% by weight of hydrocarbons other than propene, is reacted with a hydroperoxide in a solvent in the presence of a zeolite catalyst to give a mixture (G0), wherein the mixture (G0) comprises propylene oxide, solvent, unreacted propene, unreacted hydroperoxide and oxygen,
(b) the propylene oxide is separated from the mixture (G0) so as to give a mixture (G1) which comprises unreacted propene and oxygen,
(c) the mixture (G1) is brought into contact with a fluid medium comprising at least a solvent to give the mixtures (G2) and (G3), wherein the mixture (G3) comprises part of the unreacted propene and oxygen and the mixture (G2) comprises solvent and residual propene, and
wherein the mixture (G3) which has been separated off and comprises unreacted propene and oxygen has a ratio of oxygen to propene such that the mixture (G3) is not ignitable, and
wherein the mixture (G2) is fed to at least one reaction of propene with hydroperoxide, and
wherein the mixture (G2) further comprises hydroperoxide and is, in the further steps (d) and (e) following the step (c), fed to a reaction of propene with hydroperoxide, as follows:
(d) the mixture (G2) is admixed with further propene so that the propene reacts with most of the unreacted hydroperoxide present in the mixture (G2) to form propylene oxide, giving a mixture (G4), and
(e) the mixture (G4) is returned physically to the separation of step (b).

2. A process as claimed in claim 1, wherein the concentration of oxygen in the mixture (G3) is less than 12% by volume.

3. A process as claimed in either of claims 1 and 2, wherein the hydroperoxide is hydrogen peroxide and the conversion of hydrogen peroxide in (a) is in the range from 80 to 95%.

4. A process as claimed in any of claims 1 to 3, wherein the zeolite catalyst is a titanium silicalite catalyst, in particular a titanium silicalite catalyst having a TS-1 structure.

## Revendications

1. Procédé de préparation d'oxyde de propylène qui comporte au moins les étapes suivantes :
(a) du propène, contenant éventuellement jusqu'à 10% en poids d'hydrocarbures différents du propène, est mis à réagir avec un hydroperoxyde dans un solvant, en présence d'un catalyseur à base de zéolite, avec obtention d'un mélange (G0), le mélange (G0) comportant de l'oxyde de propylène, du solvant, du propène n'ayant pas réagi, de l'hydroperoxyde n'ayant pas réagi et de l'oxygène,
(b) l'oxyde de propylène est séparé du mélange (G0) de façon à obtenir un mélange (G1) qui comporte du propène n'ayant pas réagi et de l'oxygène,
(c) le mélange (G1) est amené en contact avec un milieu fluide, qui comporte au moins du solvant, avec obtention des mélanges (G2) et (G3),le mélange (G3) comportant une part du propène n'ayant pas réagi et de l'oxygène, le mélange (G2) du solvant et le reste du propène, et
le mélange (G3) séparé comportant du propène n'ayant pas réagi et de l'oxygène présentant un rapport entre oxygène et propène par lequel le mélange (G3) n'est pas inflammable, et
le mélange (G2) étant amené au moins à une réaction de propène avec de l'hydroperoxyde, et
le mélange (G2) contenant, en outre, de l'hydroperoxyde et en ce que, dans d'autres étapes (d) et (e) faisant suite à l'étape (c), le mélange (G2) est amené à une réaction de propène avec de l'hydroperoxyde de la manière suivante:
(d) le mélange (G2) est additionné de propène supplémentaire avec obtention d'un mélange (G4), le propène étant converti en oxyde de propylène par une réaction intense avec l'hydroperoxyde n'ayant pas réagi, encore présent dans le mélange (G2), et
(e) le mélange (G4) est reconduit spatialement dans la séparation selon l'étape (b).

2. Procédé suivant la revendication 1, **caractérisé en ce que** la concentration d'oxygène dans le mélange (G3) est inférieure à 12% en volume.

3. Procédé suivant l'une des revendications 1 ou 2, **caractérisé en ce que** l'hydroperoxyde est du peroxyde d'hydrogène et **en ce que** la réaction sur du peroxyde d'hydrogène dans (a) est de l'ordre de 80 à 95%.

4. Procédé suivant l'une des revendications 1 à 3, **caractérisé en ce que** le catalyseur à base de zéolite est un catalyseur à base de silicalite de titane, en particulier un catalyseur à base de silicalite de titane de la structure TS-1.
